# EUROPEAN PATENT SPECIFICATION

(11) **EP 1 252 288 B1**
(45) Date of publication and mention of the grant of the patent: **08.12.2004**
(21) Application number: 01946885.9
(22) Date of filing: 26.01.2001
(51) Int. Cl.: C12M 1/36

(54) **Method for controlling growth of brewers' yeast in a nutrient medium**
Verfahren zur Steuerung der Züchtung von Bräuhefe in einem Nährmedium
Methode de regulation de la croissance de levure de brasserie dans un milieu nutritif

(30) Priority: 28.01.2000 DK 200000134
(43) Date of publication of application: 30.10.2002
(73) Proprietor: Alfa Laval Copenhagen A/S, 2860 Soborg (DK)
(72) Inventor: ANDERSEN, Anders, DK-2830 Virum (DK)
(74) Representative: Olsen, Lars Pallisgaard
(86) International application number: PCT/DK2001/000061
(87) International publication number: WO 2001/055295

(56) References cited:
- US-A- 4 444 882
- US-A- 5 151 347
- DATABASE WPI Section Ch, Week 199206 Derwent Publications Ltd., London, GB; Class D13, AN 1992-047053 XP002152673 & SU 1 648 980 A (FOOD IND AUTOM DES), 15 May 1991 (1991-05-15) cited in the application
- PATENT ABSTRACTS OF JAPAN vol. 2000, no. 03, 30 March 2000 (2000-03-30) & JP 11 335192 A (YUKISHITSU HIRYO SEIBUTSU KASSEI RIYO GIJUTSU KENKYU KUMIAI), 7 December 1999 (1999-12-07)
- PATENT ABSTRACTS OF JAPAN vol. 009, no. 171 (C-291), 16 July 1985 (1985-07-16) & JP 60 041598 A (HITACHI KIDEN KOGYO KK), 5 March 1985 (1985-03-05)

## Description

### 1. BACKGROUND OF THE INVENTION

The present invention relates to a method for controlling the growth of an organism in a nutrient medium comprising adding to the nutrient medium one or more gaseous materials which are consumed by the organism, or which do not affect the organism; monitoring the one or more gaseous materials, or monitoring one or more gaseous materials produced by the organism; and controlling the addition of the one or more gaseous materials in response to the one or more gaseous materials to keep the growth within predetermined limits.

In particular the invention relates to such a method for controlling the cultivation or propagation of an organism, in particular yeast for brewing beer.

### The Technical Field

Organisms such as yeast grow and reproduce very rapidly in aerated nutrient medium e.g. aerated wort. The yeast cells use energy obtained from suitable nutrients e.g. carbohydrates in the wort. Aerobic organisms deplete oxygen. Once the population reaches a high density, the cells at the air-liquid interface uses all of the oxygen immediately. As a result, cells below the surface cannot grow. Aerobic metabolism of yeast cells yields very fast growth of cells which can be controlled by providing just the right amount of oxygen that the yeast cells demand. When the available oxygen has been consumed, the yeast cells adapt to the oxygen deficiency by anaerobic utilisation of the nutrients of the wort. For example the yeast cells ferment carbohydrates of the wort into various metabolites such as ethanol and carbon dioxide.

Generally, when growing organisms in a nutrient medium, both under aerobic and anaerobic conditions, it is known to control various parameters that influence the growth.

In propagation of yeast for brewing beer, for example, it is known to control the flow of sterile air added to the nutrient according to a scheme based on passed experience of the brew master to provide the desired properties of the final product produced by the propagated yeast. This method is lengthy and expensive since empirical studies of the resulting product have to be based on a batch-to-batch production basis the variability of which is difficult to control.

For use in propagation of yeast for brewing beer, e.g. lager, ale, wheat, stout, and other beers, it is known to control the parameters: content of carbon dioxide, content of oxygen, concentration of extract, temperature, and pH by measurement in the fluid of the wort to keep the growth within predetermined limits. This method has a number of drawbacks.

In propagation of yeast under aerobic conditions, the organism can be in a state where it cannot utilize e.g. the oxygen in the nutrient medium, typically because of an inhomogeneous distribution of oxygen throughout the nutrient medium. Also, the oxygen may not be available to the organism, e.g. oxygen may be bound to other molecules. Consequently, control of the growth of the organism based on measurements in the fluid of the wort, the lack of ability to use the oxygen result in a negative response of the organism to addition of oxygen based on measurements indicating excess and/or deficit of oxygen in the wort. Thus, if some parts of the wort are low in oxygen due to inhomogeneities in the oxygen distribution therein, the yeast cells may adapt themselves to the oxygen deficiency. Then, if oxygen is added, an excess of oxygen may adversely affect or "stress" the oxygen-deficiency adapted cells leading to a slower fermentation and delayed settling, e.g. of yeast for lager beer, in the fermentation process.

Other stress factors may be caused by temperature, pressure, carbon dioxide, and pH of the nutrition medium.

Further, in case of an oxygen sensor being placed in the wort, the sensor may be sensitive to other substances and e.g. proteins may clog the surfaces of the sensor and affect the accuracy and precision of the monitored oxygen. Consequently, other substances affect the control of the growth process.

Thus, for control of growth of organisms in a nutrient medium, both in batch processes and continuous processes, there is a need for an improved control of the growth of the organisms to provide organisms which are optimised for their intended application.

### Prior Art Disclosures

Anders Andersen, "Yeast Propagation Plants - Development in Relationship to History, Process and Construction", MBAA Technical Quarterly, Vol. 31, No. 2, pp. 54-57, 1994.

DE-A1-197 40 319 discloses a method and apparatus for propagating yeast cells in a brewing process. Oxygen is measured in a circulated cell suspension of a propagating tank as an indication of the oxygen excess therein.

EP-A2-0 647 708 discloses a method and apparatus for propagation of yeast in beer wort in a tank-like assimilator. The parameters of content of carbon dioxide, content of oxygen, and concentration of extract, temperature, and pH of the wort fluids measured within the interior of the assimilator. The contents of extract and oxygen is kept within limits specified for a particular propagation programme

US-A-4 764 471 discloses a method and apparatus for continuously propagating micro organisms in a medium wherein a rigorous control over the concentration of gaseous material in the medium is attainable by virtue of increased solubility of the gaseous material in the medium flowing under pressure. Use of a highly enriched gas stream and a small total volume of gas required relative to the flow of nutrient medium. Monitoring of oxygen is not disclosed.

SU-A1-1 648 980 discloses a method of automatic control of a process of culturing fodder yeast in continuous yeast fermenters, the method comprising measurement and control of a large number of parameters including temperature, pH and level of culture medium, and feed rate of inoculant yeast. The method further include additional measurements of pH of nutrient medium fed to the fermenter, pH of inoculant yeast, feed rates of air for aeration, inoculant yeast, nutrient medium, and acid for adjusting pH in the fermenter, and concentration of oxygen in gases exiting the fermenter; and additional control of feed rate of air, level and pH of culture medium, and inoculant yeast with correction of feed rate and pH of nutrient medium and inoculant yeast, and allowance for oxygen concentration in off-gasses, for increasing the yield of biomass of the microorganisms irrespectively of their specific properties. Nothing is indicated nor suggested about controlling addition of gaseous materials in response to the monitored gaseous materials to keep the growth of the organisms within predetermined limits, e.g. to keep the growth of organisms with desired properties, such properties depending e.g. on the amount of oxygen available.

US-A-4 444 882 discloses a process of controlling cultivation of microorganisms in culture medium in a cultivation tank for maintaining better substrate supply conditions or maintaining high yield of product, said process comprising measuring concentration of carbon dioxide in the effluent during cultivation and supplying additional medium in accordance with the calculated amount of propagated microorganisms based on the amount of carbon dioxide produced. Further, aerobic cultivation is controlled by controlling the concentration of dissolved oxygen, said concentration of dissolved oxygen being controlled by at least one of (a) aeration rate of oxygen-enriched gas, (b) concentration of oxygen in the oxygen enriched gas, and (c) revolution of a stirrer.

US-A-5 151 347 discloses controlled production of mircoorganisms by photosynthesis in a closed photobioreactor specially designed to operate with expensive carbon isotopes as reactant gas in a closed loop. Oxygen is produced as a waste product and removed from the gas mixture in a combustion reaction e.g. with hydrogen in a catalytic converter. Oxygen is measured in the gas phase or liquid phase of the biosynthetic culture, and when a predetermined level is exceeded, hydrogen is caused to flow into the catalytic converter (column 3, line 61 to column 4, line 13). Hydrogen is measured in order to avoid introducing an excessive amount of hydrogen into the catalytic converter (see column 4, lines 14-19).

JP-A-11 335 192 discloses a method and apparatus for composting solid biomass waste by aerobic fermentation using ripe compost as seed bacteria, i.e. converting said solid biomass waste into a mixture of decaying organic substances used for fertilizing land, wherein the oxygen concentration in the exhaust gas is measured and used to control the oxygen concentration in an oxygen-containing gas, here air, and the quantity thereof.

JP-A-60 041 598 discloses a fermentation process for composting organic waste such as sewage sludge wherein the concentration of oxygen is measured in the waste gas and the set value of the quantity of air passed is adjusted based on the total quantity of consumed oxygen.

### 2. DISCLOSURE OF THE INVENTION

### Obiect of the Invention

It is an object of the present invention to seek to provide an improved method for controlling growth of an organism in a nutrient medium.

In particular, it is the object to seek to provide an improved method for controlling growth of an organism which organism is suited for production of specific products.

More particular, it is the object of the present invention to seek to provide such a method for improving the control of propagation of yeast, in particular yeast for brewing beer.

It is still another object of the present invention to seek to provide means for optimising the operation and control of such a method.

Further objects will appear from the description elsewhere.

### Solution According to the Invention

### "Method of controlling growth of an organism in a nutrient medium"

In an aspect of the invention these objects are fulfilled by providing a method of controlling growth of an organism in a nutrient medium as claimed in claim 1 wherein the one or more added and/or produced gaseous materials are monitored in the exhaust gas of the nutrient medium.

It has surprisingly turned out that when the one or more added and/or produced gaseous materials are monitored in the exhaust gas of the nutrient medium, as opposed to monitoring said one or more added and/or produced gaseous materials within the nutrient medium, the control of the addition of the one or more gaseous materials in response to the one or more of the monitored gases to keep the growth of the organism within predetermined limits is improved.

### Monitoring in the exhaust gas of the nutrient medium

According to the present invention, the one or more gaseous materials are monitored in the exhaust gas of the nutrient medium.

The exhaust gas of the nutrient medium can be monitored at any suitable location outside of the nutrient medium.

In a preferred embodiment, a suitable location is one wherein an integral value of the one or more gaseous materials can be measured. This can be obtained by mixing the contributions from various locations in the nutrient medium, and then monitor the one or more gaseous materials in a common exhaust gas of the mixed gases in one or more outlets. This embodiment is particularly useful in a continuous growth process with a flowing medium e.g. in a plug flow reactor.

In another embodiment, suitable locations are locations wherein individual values of the one or more gaseous materials can be measured of the exhaust gas of the nutrient medium by avoiding one or more gaseous materials from the various locations.

In a preferred embodiment, the nutrient medium is contained in an essentially closed container with at least one outlet for the exhaust gas wherein the one or more added and/or produced gaseous materials are monitored in said at least one outlet for the exhaust gas outside the container whereby it is obtained that an integral value of the one or more monitored gaseous materials is obtained.

Further, it is obtained that foam optionally present in the headspace of the container above or in the interface of the nutrient medium does not influence the sensor of the monitored gaseous material.

Also, cleaning and sterilization of sensors to be inserted into the nutrient medium is avoided.

In another preferred embodiment, the one or more added and/or produced gaseous materials are monitored in the exhaust gas in one or more locations of the headspace of the container above the nutrient medium whereby it is obtained that individual values for each location are obtained and that the distribution of the added gaseous material can be monitored. This is particularly useful in a continuous growth process with a flowing medium.

In still a further preferred embodiment, the one or more added and/or produced gaseous materials are monitored in both in said at least one outlet of the exhaust gas outside the container and in said one or more locations of the headspace of the container above the nutrient medium whereby the combinatorial effect of simultaneously obtaining both integral and individual values of the one or more monitored gaseous materials is obtained.

### Response to the monitored one or more gaseous materials

Generally, the various parameters used to control the growth of an organism in a nutrient medium may be adapted in response to the monitored parameters in order to keep the growth within predetermined limits.

In a preferred embodiment, the control of addition of the one or more gaseous materials is adapted in response to a decrease and/or an increase in one or more of the monitored gaseous materials, or a derivative thereof, whereby it is obtained that the added gaseous material is adapted to the one or more gaseous materials which have been made available to the organism, or which the organism has produced. This is different to the previous methods where the gaseous material is added in response to the monitored one or more gaseous materials available to the organism in a nutrient medium.

Thus, assuming that the organism has been presented to a sufficient amount of a particular gaseous material, the gaseous material, which is not used by the organism, appears in the exhaust gas. Consequently, controlling the addition of the one or more gaseous materials in response to desired levels of the one or more gaseous materials in the exhaust gas, it is ensured that the amounts of gaseous materials are always sufficient to cover the demand of the organism.

It should be noted that the levels of the one or more gaseous materials in the exhaust gas can be selected as desired to expose the organism to certain stress factors, if so desired.

Various levels of stress to the organism can be obtained. If the addition of a certain gaseous material provides a small value of the gaseous material in the exhaust gas, the stress level is low. It can even be negative if the presence of the gaseous material is essential to the organism. However, if a high level of the gaseous material is present in the exhaust gas, more gaseous material is made available to the organism than it requires which may consequently stress the organism.

In a preferred embodiment, the control of the addition of one or more gaseous materials is further adapted in response to one or more constant levels of one or more other monitored gaseous materials whereby especially addition of one or more gaseous materials in the start up phase can be controlled.

An example of this being the combined addition of air and pure oxygen, the latter of which is typically the most expensive of the two, wherein the level of added pure oxygen may be adapted in response to a monitored constant level of e.g. nitrogen thereby ensuring an optimum use of the more expensive pure oxygen and the cheaper air.

### Gaseous materials

The one or more gaseous materials which the organism consumes, or which do not affect the organism, includes gaseous materials which the organism absorbs and which participate in the metabolic processes, either directly or indirectly. Especially, the added gaseous materials may be fully or partially involved in these processes. Also, all or a part of the added gaseous materials may be consumed.

Thus, these gaseous materials include primary metabolites that are involved directly in the growth of the organisms through the growth phase, and gaseous secondary metabolites that are produced in the stationary phase of the growth.

In a preferred embodiment, the one or more gaseous materials which are consumed by the organism, or which do not affect the organism, is selected from the group consisting of oxygen, carbon dioxide, and nitrogen, and mixtures thereof.

### Monitored quantity

Generally, the monitored gaseous materials are measured in any suitable quantity including concentration and flow, and any suitable derivative thereof, either based on moles, weight or volume.

In a preferred embodiment, the response of the one or more of the monitored gaseous materials is measured in concentration of gaseous component with respect to the total gaseous material.

### Signals of the monitored one or more gaseous materials

Generally, the signals of the one or more monitored gaseous materials comprises a constant level, a decreased level, and an increased level, or a combination thereof.

For example, a decrease in the level of monitored oxygen may be monitored, simultaneously with an increase of monitored carbon dioxide and a constant level of monitored nitrogen.

For various phases of the growth process, the skilled person can contemplate other combinations.

### Other parameters such as temperature and pressure

Generally, the growth of an organism in a nutrient medium is influenced by a large number of parameters.

In a preferred embodiment, the addition of the one or more gaseous materials is adapted in response to the temperature of the nutrient medium whereby it is obtained that the stress level of the organism is controlled depending on the rate of growth of the organism. Typically, the rate of growth of an organism is higher at a higher temperature.

In another preferred embodiment, the addition of the one or more gaseous materials is adapted in response to the pressure of the nutrient medium whereby the influence of e.g. the hydraulic pressure in the nutrient medium on the control of the addition of the one or more added and /or produce gaseous materials can be accounted for.

For example the one or more gaseous materials may be added to the nutrient medium at different positions in the nutrient medium, e.g. at different heights of a propagation tank, depending on the hydraulic pressure at the position.

### Batch and continuous processes

Generally, the method according to the present invention can be carried out in any suitable process, including batch process, continuous processes, or semi-continuous processes.

In a preferred embodiment, the method is carried out in a batch process.

In another preferred embodiment, the method is carried out in a continuous process.

### Organisms

The organism to be grown according to the present invention can be any organism that can grow in a nutrient medium. This includes organisms that can grow both under aerobic and anaerobic conditions.

In addition to metabolic processes of producing CO₂, the metabolic processes may include both oxidative respiration processes producing reduced inorganic gaseous products such as N₂O, N₂, H₂S, and CH₄ and fermentation processes producing reduced organic products such as CH₄. The growth may be measured both by the number of cells and by the size of single cells.

In preferred embodiments the organisms include bacteria and fungi. Examples of organisms are Saccharomyces, Streptococcus, Lactobacillus, and Penicillium. Specific strains of Saccharomyces include S. carlbergensis for bottom fermentation of e.g. lager beers and S. serevisiae for both bottoms and top fermentation of other beers..

In a particularly preferred embodiment the organism is yeast for brewing beer.

### Predetermined limits

Generally, for a given organism and its desired application, it is known to try to keep the growth of the organism within predetermined limits from a desired growth, e.g. the growth curve falls within desired limits. In the present context, the term "predetermined limits" is not limited to number of cells per time unit in the growth curve but may include conditions of the growth which provide organisms with desired properties. Such organisms may exhibit a desired viability and vitality. The growth conditions may also include the aim to produce a large number of living organisms suited for particular applications such as the production of specific products including e.g. specific flavours and smelling substances. Or the growth conditions may include aiming at producing a culture of organisms that maintain anaerobic fermentation characteristics after the cells have been subjected to aerobic growth. This applies to facultative organisms in particular yeast for brewing beer.

Predetermined limits can include growth conditions that produce organisms that exhibit no stress symptoms, stress symptoms, or a combination thereof depending on the desired application of the organism.

Predetermined limits are determined based on empirical studies, including microscopy of cells for number, shape and size.

### "Apparatus for growing an organism in a nutrient medium Suitable for carrying out the method of the invention"

The apparatus comprises an essentially closed container, at least one gas additions means, at least one exhaust gas means, at least one process monitoring means for monitoring one or more parameters of the growth process comprising at least one gas monitoring means of gaseous materials, and at least one process control means, as claimed in claim 14, wherein the at least one gas monitoring means is adapted to monitor the one or more gaseous materials in the exhaust gas of the nutrient medium.

### Gas monitoring means

The at least one gas monitoring means may be adapted to monitor the one or more gaseous materials in the exhaust gas of the exhaust gas means outside the container, or to monitor the one or more gaseous materials in the headspace of the container above the nutrient medium, or to monitor the one or more gaseous materials both in the exhaust gas of the exhaust gas means outside the container and in the headspace of the container above the nutrient medium.

Preferably, the gas addition means include means for providing small or large bubbles, or mixtures thereof, whereby the gas addition can be further adapted to the addition and distribution of gaseous material.

### Gas addition means

The gas addition means may be adapted to add one or more gaseous materials in response to a decrease and/or an increase in the one or more of the monitored gaseous materials, or a derivative thereof, in the exhaust gas of the nutrient medium.

The gas addition means may be further adapted to add one or more gaseous materials in response to one or more constant levels of the one or more monitored gaseous materials.

Preferably, the gas addition means include means for providing small and large bubbles, or a mixture thereof, whereby the gas addition can be further adapted to the addition and distribution of gaseous material.

### Exhaust gas means

The exhaust gasses of the nutrient medium are guided away from by any suitable means.

It is preferred that the exhaust gas means are adapted to collect the exhaust gas in the headspace above the nutrient medium and to discharge the exhaust gas through a common outlet whereby the exhaust gas generated in various locations of the nutrient medium is mixed and whereby a monitored gaseous material is monitored for an integral value of the total exhaust gas.

Alternatively the exhaust gas means may be adapted to let out the exhaust gas through individual outlets from different locations above the nutrient medium whereby the exhaust gas from various locations are kept as individual partially mixed exhaust gases.

In other examples, the exhaust gas means are adapted to both collect the exhaust gas in the headspace and to discharge the exhaust gas through a common outlet and to let out the discharge gas through individual outlets from different locations above the nutrient medium.

### Gas monitoring means

The one or more gaseous materials can be monitored by any suitable monitor known in the art, either in-line by continuous monitors, or off-line by sampling of suitable samples and subsequent measurement with suitable analytical equipment, e.g. gas chromatograph.

Monitors of gaseous materials comprise monitors for gases such as oxygen, carbon dioxide, and nitrogen but are not limited hereto. Other gas monitors include monitors for e.g. methane.

The gas monitors can be any monitor based on any suitable measuring principle that relates a response to the concentration or flow of the gaseous material in question, or any other suitable quantity.

Suitable gaseous monitors for e.g. oxygen monitors are ceramic oxygen monitors.

### Addition and distribution of gaseous material

The one or more added gaseous materials may be added to the nutrient medium in any suitable way. This includes addition of said gases at different heights of the nutrient medium as well as at different position within a certain height.

Preferably the apparatus further comprises means for distribution of the one or more gaseous materials in the nutrient medium whereby the gaseous material can be more homogeneously distributed in the nutrient medium.

Gas distribution means comprises gas nozzles, gas distribution conduits, and agitation means, but are not limited thereto.

### Process control means for temperature and pressure

The process control means comprises temperature controlling means for controlling the temperature of the nutrient medium and the at least one process monitoring means comprises temperature monitoring means, or pressure controlling means and at least one process monitoring means for controlling the pressure within the container.

### Process control means

The process control means of the apparatus include any suitable computing means which is able to control the growth of an organism by receiving measuring signals from the at least one process monitoring means, including signals from gaseous monitors, flow monitors, temperature and pressure monitors, and by transmitting control signals to various flow valves and pressure valves to control flows of various process streams and pressures, respectively, of the gas addition means and exhaust gas means.

Preferably the process control means comprises a computer whereby the sequence of process monitoring and controlling steps can be easily executed.

Thus, the method of the present invention can be carried out by providing a computer program for controlling the growth of an organism in a nutrient medium in an apparatus as described previously.

Preferably, the computer program controls a process control means for controlling one or more parameters of the growth process in an apparatus according to the invention.

Particularly preferred is when the computer program is adapted to control the addition of gaseous material in response to the monitored one or more gaseous materials.

### 3. BRIEF DESCRIPTION OF THE DRAWINGS

In the following, the invention is further disclosed with detailed description of preferred embodiments by way of examples only, reference being made to the drawings in which
Fig. 1 shows a sketch of an apparatus for growing an organism in a nutrient medium according to the prior art, here illustrating the principle of propagation of yeast for brewing beer,
Fig. 2 shows a side view sketch of an apparatus for controlling growth of an organism in a nutrient medium according to the invention,
Fig. 3 shows an apparatus as shown in Fig. 2 incorporated in a system for propagation of yeast for brewing beer,
Figs. 4A and 4B show schematic diagrams of an example of a process monitoring means and process control means for monitoring one or more parameters of the growth process and for controlling these parameters in response to the monitored values thereof, and
Fig. 5 shows a plot of experimental results for a growing process in a preferred embodiment.

### 4. DETAILED DESCRIPTION

### "Propagation of brewing yeast"

The production (propagation) of brewing yeast is typically based on batch processes in closed containers under strict sanitary conditions. The purpose is to produce of a certain amount of yeast with good fermentation characteristics which to some degree is counteracting yeast growth because yeast growth is obtained primarily under aerobic conditions and fermentation is an anaerobic process.

Under these conditions, the optimal condition for brewing yeast propagation is to supply only as much oxygen as the yeast is able to take up without damaging the fermentation characteristics (oxygen stress) of the yeast.

According to a preferred embodiment, to ensure the correct supply of oxygen, oxygen is measured in the exhaust gas leaving the nutrient medium. If oxygen is registered in the exhaust gas leaving the nutrient medium under optimal air-transfer conditions, it is because the yeast has not been able to utilise the oxygen.

The typical course of oxygen consumption during propagation of brewing yeast is as follows:

### 1. Start phase

Very low oxygen consumption because small amount of yeast and high concentrations of monosaccharides (mainly glucose) are present. Monosaccharides suppress aerobic metabolism to a much higher degree than disaccharides (mainly maltose). Nonetheless the yeast prefers monosaccharides and these are consumed before the disaccharides.

### 2. After monosaccharides are consumed

After a period the monosaccharides are consumed and this gives way to aerobic metabolism and the oxygen uptake - and therefore also the yeast mass - grows.

### 3. After growth stops

The logarithmic growth period stops after some time - most likely due to the fact that the substrate runs out of growth substances, either free amino nitrogen or others, but it may also be because when placed in a concentrated sugar solution only a certain number of cell divisions are possible - and the oxygen consumption therefore declines.

The oxygen consumption thus varies considerably in the period of propagation and in all phases it is important not to overdose the oxygen supply in order not to give stress to the yeast which may cause suboptimal fermentation characteristics.

The control system can involve not only an oxygen monitor but also a carbondioxide monitor in the exhaust gasses.

A certain pressure is maintained in the tank during the propagation in order to avoid infections coming from outside and to avoid foaming and controlling the pressure of the gaseous materials. The carbondioxide is toxic to the yeast and it is therefore important to minimize the content in the tank. When the oxygen uptake and yeast growth stops, the carbondioxide production declines and the foam problems therefore become less pronounced. In the end of the propagation period it is therefore beneficial to minimize the pressure in order to get as much carbondioxide as possible out of the solution. With signals from the oxygen and carbondioxide monitors indicating an increased oxygen concentration and a decline in carbondioxide concentration in the exhaust gasses, the overpressure is minimized, the propagation is finished and the yeast ready to be used for fermentation.

### "Prior art apparatus"

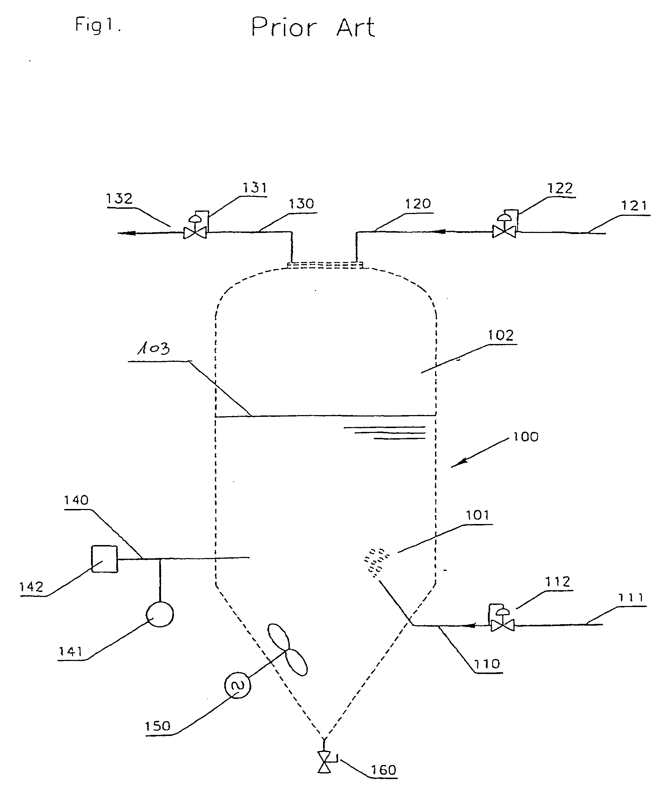

Fig. 1 shows a sketch of an apparatus for growing of an organism in a nutrient medium according to prior art, here illustrating the principles of propagation of yeast for brewing beer.

The apparatus comprises an essentially closed container; here a tank 100 comprising an upper part 102 and a lower part 101 with a conically shaped bottom. The container contains the organism, the nutrient medium, added gaseous materials, and materials produced by the organism as a result of its metabolic activities, here the organism is yeast for brewing beer either top-fermenting yeast or bottom-fermenting yeast. The nutrient medium is wort which is obtained by methods known in the art, usually obtained by separating the liquid phase from the resulting suspension in the marking process. The wort is placed in the lower part 101 of the tank forming a more or less well defined interface region 103 with gaseous material in the upper part of the tank. The interface region may include a region with a foamed phase of the wort. The gaseous material provided by the organism is primarily carbon dioxide that is collected in the headspace of the upper part 102 of the tank.

The apparatus comprises at least one gas addition means for adding one or more gaseous materials into the container. Here two gas addition means are shown. A first gas addition means includes a gas inlet 110 allowing pressurised sterile air supplied through an air supply connection 111 to be conducted via a control valve 112 to the lower part of the tank. Typically, the tank further comprises a stirring means 150 for distributing and mixing the added gaseous material with the various components of the wort, said stirring means being operable from the outside of the tank. A second gas addition means includes a gas inlet 120 of sterile air supplied through an air supply connection 121 at the top of the tank used in the process of sterilisation and emptying of the tank. Here sterile air is supplied through another air control valve 122, optionally connected to an air supply source common with the first air supply connection 112. The second inlet of sterile air is supplied in the upper part of the tank to the top of the wort either for addition of gaseous material for the growth of the organism and/or for controlling the pressure in the headspace of the tank.

The apparatus further comprises at least one exhaust gas means for discharging exhaust gas of the nutrient medium from the container. Here the tank is equipped with an exhaust outlet 130 for discharging gases in the top of the tank, typically said exhaust outlet includes a back pressure control valve 131 for controlling the pressure in the tank and for discharging the exhaust gas in the outlet conduit 132.

The apparatus further comprises at least one process monitoring means for monitoring one or more parameters of the growth process comprising at least one gas monitoring means for monitoring the gaseous materials of the one or more added gaseous materials, and/or at least one gas monitoring means for monitoring other gaseous materials produced by the organism. Here the gaseous monitoring means consists of a sampling tube 140 for taking samples in the wort within the tank. Samples are subsequently analysed typically off-line by suitable analytical equipment 141, here shown logically coupled to the sampling tube. On-line analysis can also be done. Sampling of the wort can be for monitoring various parameters of the nutrient medium such as pH, and temperature, and various concentrations of e.g. oxygen and carbon dioxide as well as yeast cells within the tank. Alternatively, the gaseous monitoring means can include monitoring probes placed in the wort (not shown).

The apparatus further comprises a process control means for controlling the one or more parameters of the growth process in response to outputs of the at least one process monitoring means. Here a computer 142 is shown logically connected to the sampling tube of the process monitoring means.

The apparatus also includes an inlet/outlet valve 160 for adding nutrient medium to the tank and for removing the produced product therefrom.

The apparatus can include other means and devices (not shown) e.g. cleaning means such as spray balls for cleaning the tank between batches; heating means and cooling means for regulating the temperature of the wort, tank, and conduits.

According to this prior art control of the growth of an organism added to the nutrient medium is obtained by aeration of the wort by adjusting the air control valve 112 until a suitable flow of sterile air is produced in the tank. The parameters of carbon dioxide, oxygen, concentration of extract, temperature and pH are measured in the wort by means of the sampling tube 140, the analytical equipment 141 and the computer 142. Exhaust gases of nitrogen, carbon dioxide and excess of oxygen are discharged at the exhaust valve 131 into the outlet conduit 132.

The exact criterion for a suitable flow of sterile air is based on the individual wort. Further, it is based on the empirical experience of the brew master to adjust the aeration to keep the growth within predetermined limits.

In case of brewing beer, the experience of the brew master in propagation of the yeast is decisive to obtain the desired properties of the final beer product e.g. with respect to the amount of alcohol and other substances such as aroma substances and taste providing substances.

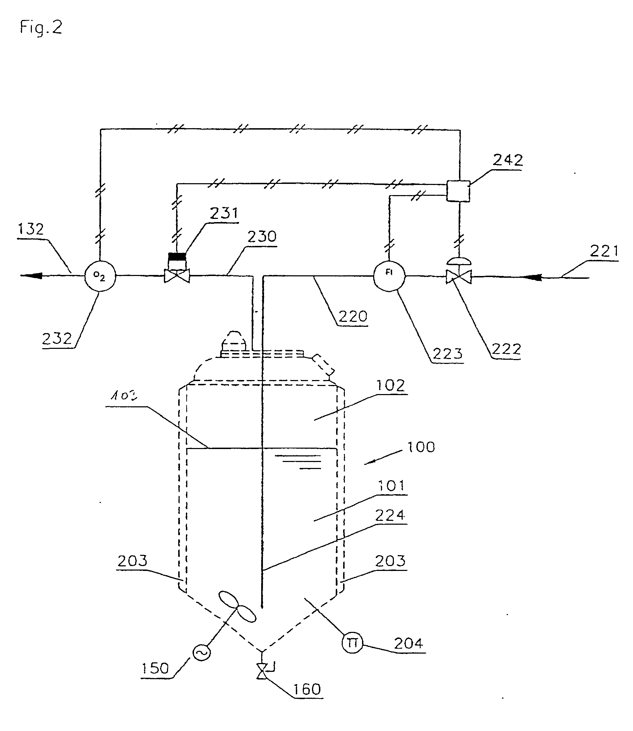

Fig. 2 shows a side view sketch of an apparatus for controlling growth of an organism in a nutrient medium according to the present invention.

The apparatus comprises an essentially closed container for containing the organism, the nutrient medium, added gaseous materials, and the materials produced by the organism as a result of its metabolic activities. The container is a tank similar to that shown in Fig. 1 having a lower part 101 and an upper part 102.

Preferably, a heating/cooling jacket 203 surrounds the container. A temperature sensor 204 is located in the lower part of the tank, preferably near to the inlet 224.

The apparatus comprises at least one gas addition means for adding one or more gaseous materials into the container. Here one gas addition means is shown including a gas inlet 220 allowing pressurised sterile air supplied through an air supply connection 221 to be conducted via a control valve 222 and flow meter 223 to the inlet 224 in the lower part 101 of the tank. Typically, the tank further comprises a stirring means 150 for distributing and mixing the added gaseous material with the various components of the wort, said stirring means being operable from the outside of the tank.

The apparatus further comprises at least one exhaust gas means for discharging exhaust gas of the nutrient medium from the container. Here the tank is equipped with an exhaust outlet conduit 230 for discharging gases in the upper part 102 of the tank, typically said exhaust outlet includes a back pressure control valve 231 for controlling the pressure in the tank. The outlet conduit 230 is shown to let out gasses in the upper most part of the upper part 102 of the tank thereby providing an integral of the discharged gasses across the tank. However, the point of outlet in the tank can be selected from one or more other locations in the headspace of the container above the nutrient medium. Also, gas traps in optional foam of the nutrient medium can be a point of outlet.

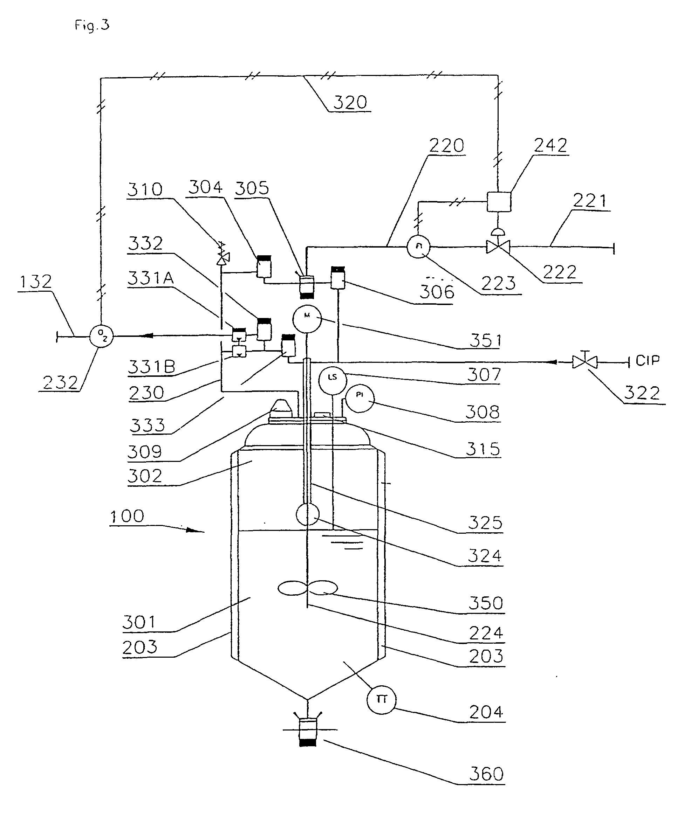

The apparatus further comprises at least one process monitoring means 232,223 for monitoring one or more parameters of the growth process comprising at least one gas monitoring means 232 for monitoring the gaseous materials of the one or more added gaseous materials, and/or at least one gas monitoring means for monitoring other gaseous materials produced by the organism. Here the gaseous monitoring means include an oxygen meter 232 for monitoring the oxygen in the outlet conduit 132 and a flow meter 223 for measuring the flow of sterile air. Other monitoring means include means for monitoring pressure (e.g. 308 as shown in Fig. 3) and temperature 204.

The apparatus further comprises a process control means for controlling the one or more parameters of the growth process in response to outputs of the at least one process monitoring means. Here a computer 242 is shown logically connected to the oxygen meter 232 and the flow meter 223. Further the computer is connected to valves 222 and 231.

The apparatus also includes an inlet/outlet valve 160 for adding nutrient medium to the tank and for removing unused nutrient medium including the produced product there from similar to that of the prior art shown in Fig. 1.

The computer is programmed in any suitable programming language to perform monitoring and control functions of the various parameters, including pressures, temperatures, and flows of the respective sensors and by transmitting signals to the respective control valves, heaters/coolers, etc.

Generally, the computer is programmed to control the flow of gaseous material added, here sterile air added to the tank in response to the flow of exhaust gasses, here oxygen leaving the tank.

Preferably, the computer is programmed to control the addition of sterile air until the flow of oxygen in the outlet is close to zero. This condition is typically kept through out the growth process by increasing/decreasing the flow of the added sterile air. After saturation, the flow of sterile air is typically low at the beginning and adapted so that the organisms in the nutrient medium absorb the added one or more gaseous materials, here oxygen of the sterile air. Then the flow of sterile air increases to further adapt the amount of oxygen so that the organisms absorb that added. After the growth rate of the organisms has reached its maximum, the flow of sterile air then decreases and finally stops as the demand for oxygen by the organisms levels off.

Alternatively, the computer is programmed to control temperatures over time and in response to the values of the monitored parameters whereby it is obtained that the temperature conditions for the organism can be controlled. In particular it is obtained that the combined response of both the addition of gaseous material and the increase/decrease of temperature can be controlled thereby ensuring a particular efficient control of the growth of the organism.

In this example, for controlling the temperature, the heating/cooling jacket 203 is controlled in response to the temperature transducer 204.

In still another example, the computer is programmed to monitor and control the pressure in tank.

Fig. 3 shows the embodiment in Fig. 2 incorporated in a system for propagation of yeast for brewing beer.

Compared to the system of Fig. 2, this example further includes means which are particular useful in further improving the control of the growth process.

Such means include distribution means. Here, the distribution means comprises a rotor means, such as a propeller 350, a rotor connection means, here a tube 325 including a rotary shaft, e.g. a rod or cylindrical shaft (not shown), and a motor, here a computer controlled externally driven motor 351. The distributor means is connected via the distribution connection means to the externally driven motor for agitation of the wort in the tank. The distributor means is preferably placed centrally in the tank thereby allowing the site of agitation in the nutrient medium to be centrally placed near the inlet 224 of the added gaseous material, here sterile air, in the lower part of the tank 301. However, the site of agitation, or optionally more sites of agitation, can be chosen in other parts of the tank, e.g. in order to provide suitable agitations of added gaseous materials through more inlets thereby obtaining a more homogeneous distribution of the components. A more efficient control of the agitation, the mixing, and the distribution of the added gaseous material, wort, and organisms in the wort is obtained which provides a further improvement of the control of the growth process.

For the purpose of cleaning the interior of the tank, the valves, and conduits for contaminating organisms, the apparatus further comprises a cleaning means for so-called cleaning-in-place operation that is particularly useful in combination with the process monitoring means, in particular the gas monitoring means. The cleaning-in-place means comprise means for supply 322 (CIP) of cleaning fluid, here a hot caustic solution. Further, it comprises means for closing the conduit 220 for adding gaseous material, here a pneumatic leak proof T-valve 305 coupled to the CIP supply through a pneumatic angle valve 306 and coupled to the outlet conduit 230 through another pneumatic angle valve 304. The top pressure conduit is further connected to a safety valve 310 in case of overpressure.

In operation during cleaning of the conduit 224 for adding gaseous material to the tank, here the lower part of the tank, the T-valve 305 is arranged to close for passage of sterile air from the conduit 220 and open for passage of the cleaning fluid supply CIP through the pneumatic valve 306 and down into the tank through the inlet 224. The pneumatic valve 304 is normally closed during this phase. If it is open, the cleaning fluid can pass directly to the top pressure conduit 230 in order to clean this line.

Also, during cleaning, the distribution connecting means 325 comprises a passage for cleaning fluid from the CIP supply to a spray ball 324. The spray ball may be mounted in fixed or variable position to provide a spray of cleaning fluid in a desired spray pattern and level of the tank. During cleaning the pneumatic valves 333 and 332 are pulsating to allow the cleaning fluid to pass to the exhaust outlet 132 through the backpressure control valve 331 a. Furthermore, cleaning fluid passed through anti vacuum valve 331 b to the top pressure conduit 230.

In operation of the apparatus during growth of the organism and addition of sterile air, the exhaust gas collected in the upper part of the tank 302 is discharged through the back pressure control valve 331 a and top pressure conduit 230 while the pneumatic angle valves 333, 332, and 304 and the safety valve 310 are closed. Leak-proof valve 305 and angle valve 306 are open.

The backpressure control valve 331 a can for example be a pressure exhaust valve of the type X4.27.39.100 supplied by Alfa Laval Scandi Brew, Søborg, Denmark.

The addition of sterile air is continuous in the amount needed by the growth process.

For visual inspection, the tank also comprises a tank light 309 and a tank sight glass 315 whereby the growth of the organism can be visually inspected by manually viewing the foam and evaluating the growing process for its characteristics, e.g. the characteristic appearance of the foam for unstressed conditions of the yeast.

A high-level probe 307 and pressure monitor here a pressure gauge 308 are mounted on the top of the tank.

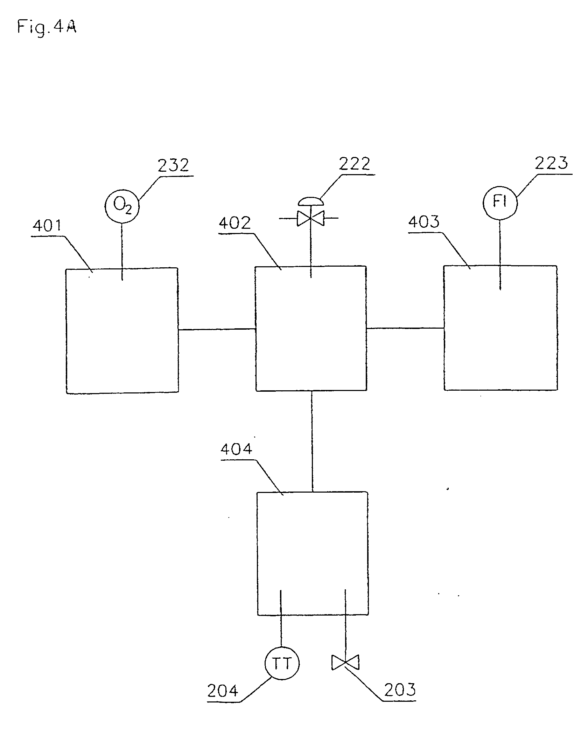

Fig. 4A shows a schematic diagram of an embodiment of a process monitoring means and process control means, here generally referred to by reference numeral 424, for monitoring one or more parameters of the growth process and for controlling these parameters in response to the monitored values thereof.

The process monitoring means comprises an oxygen meter 401 (or meter for other gases) logically coupled to an oxygen sensor 232 (or sensors for other gases) and to a process controlling means here a computer 402.

In a preferred embodiment, the process monitoring means comprises a carbon dioxide meter for monitoring carbon dioxide coupled to a carbon dioxide sensor (not shown).

The carbon dioxide sensor is positioned at a similar location as the oxygen sensor.

Similarly the process monitoring means comprises a flow meter 403 logically coupled to a flow meter 223 and to the process controlling means, also here a computer 402.

Also, the process monitoring means comprises a temperature controller including another process control means, here another computer 404, and logically coupled to a temperature transducer 223 and to a heating/cooling jacket 203. Further, the two process-controlling means, i.e. the computers 402 and 404, are logically coupled.

It is preferred that the process monitoring means comprises further monitors e.g. a level monitor connected to a level detector 307 e.g. a level switch LS, and a pressure monitor connected to a pressure sensor e.g. a pressure transducer PI 308.

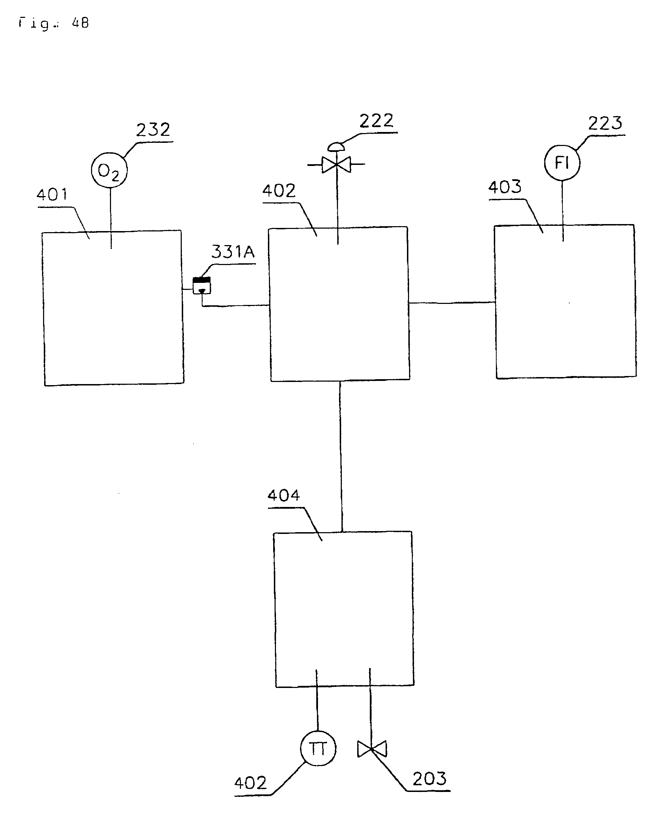

FIG. 4B shows an example wherein the back pressure control valve 331 a is controlled.

### 5. EXAMPLE

The invention is further illustrated by an example of controlling the growth of yeast cells (lager yeast).

### Procedure

A tank equipped as described in connection with figs. 2 was filled with about 20 hl nutrient medium, in form of aerated standard wort with 14 degree plato. Any equivalent wort or suitable nutrient can be used. The wort was previously sterilised and cooled to a fermentation temperature of initially 15-16 °C.

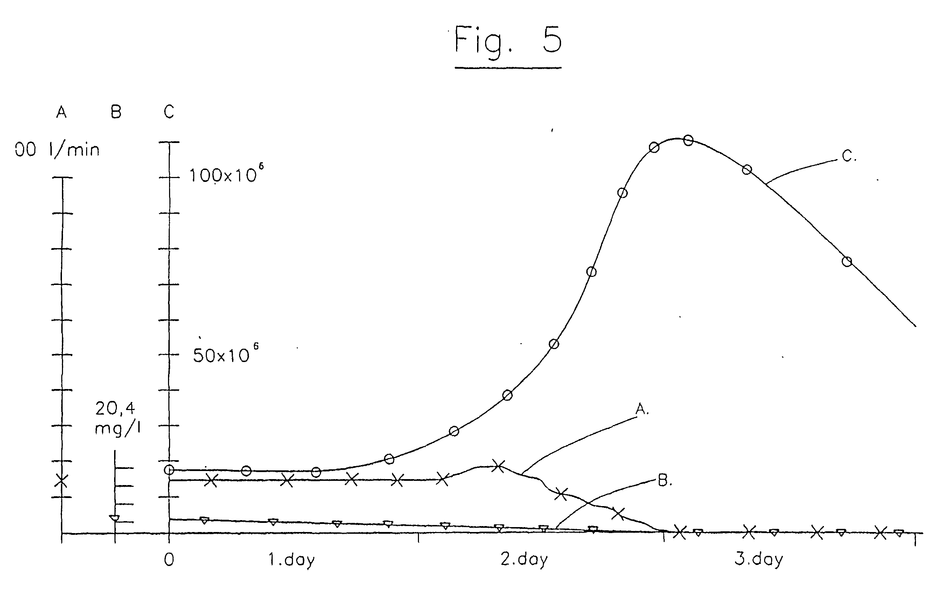

A sufficient amount of sterile CO₂ was let through the nutrient medium under gentle agitation in order to drive off non-dissolved O₂ from the medium and oxygen in the headspace. (See Fig. 5 curve B at zero time origin).

After this the oxygen concentration in the headspace was 5 mg O₂ per liter.

20 liter second generation Tuborg yeast cells (a variety of S. carlsbergenesis) were inoculated into the wort through a sample cock (not shown).

Sterile air was added by adjusting the air supply valve 222 to provide a desired sterile air flow rate (curve A of Fig. 5) corresponding to 20 I sterile air per minute as measured by the flow indicator 223.

The reading on the oxygen meter 232 was recorded showing a slight decline above zero (Curve B on Fig. 5)

At the beginning of the second day analysis showed an increased cell count. Further to maintain the oxygen concentration in the outlet 132 within a range of above zero and below 1 mg O₂ per liter, the amount of sterile air was increased (end of first day to start of second day). The demand for oxygen increased for the increasing number of cells curve C.

Following a propagation phase of usually 3-7 days depending on the yeast cell, the high kraussend stage was reached, here 2,5 days and the propagation process was stopped by terminating the addition of sterile air.

The number of cells (curve C of Fig. 5) was measured by sampling and measurement using a standard cell counting apparatus.

For comparison, growth experiments carried out according to the method of the present invention have shown a much better control over prior art methods could be obtained.

Further, the quality of the cells was investigated by microscopy and indicated healthy unstressed yeast cells.

Also, a considerably smaller number of dead cells was obtained.

### A brew master's recipe - propagation example - explanatory description

An embodiment of the invention is further illustrated by example of a brew master's recipe which is summarized in the subsequent table.

### Step 1 - Wort sterilisation

The wort is sterilised by supplying steam to the heating jacket on the tank cone. Heating continues until the wort has reached a temperature of 120 °C which is held for 30 minutes. In order to reach this temperature and avoid boiling, pressure is increased to 1.5 bar G (over pressure).

### Step 2 - Wort cooling

After sterilisation steam supply to the cone is stopped and cooling through the cooling jacket of the tank cylinder is started. When temperature reaches 25 °C, aeration is started in order to supply oxygen to the wort and - as a secondary effect - to agitate the tank content thereby reducing the cooling time as the temperature gradient becomes smaller. Pressure is reduced to 0.5 bar G. Cooling continues until propagation temperature is reached (e.g. 20 °C).

### Step 3 - Inoculation

The tank is inoculated with yeast from the laboratory grown in a Carlsberg flask. Temperature and pressure are maintained at propagation conditions (see step 4), no aeration is performed.

### Step 4 - Propagation

During propagation temperature is maintained at a fixed temperature (e.g. 20 °C) and pressure 0.5 bar G. Aeration is performed by interval aeration and aeration is stopped when oxygen is registered in the exhaust gas indicating that more oxygen is supplied than the yeast can take up.

### Step 5 - End of propagation

When propagation period is approaching the end two events occur:
1. The period between aeration start and registration of oxygen in the exhaust gas becomes shorter (e.g. 30 seconds) indicating that the yeast does not take up more oxygen.
2. A reduction in carbondioxide production is registered by the carbondioxide monitor.

When one of these two events occur it is a signal that yeast growth is declining and propagation period is coming to an end. At this stage a signal is given to the back pressure control valve to reduce pressure to 0.2 bar G in order to minimize the amount of carbondioxide dissolved in the tank. The carbondioxide is toxic to the yeast the content of dissolved carbondioxide must therefore be minimized.

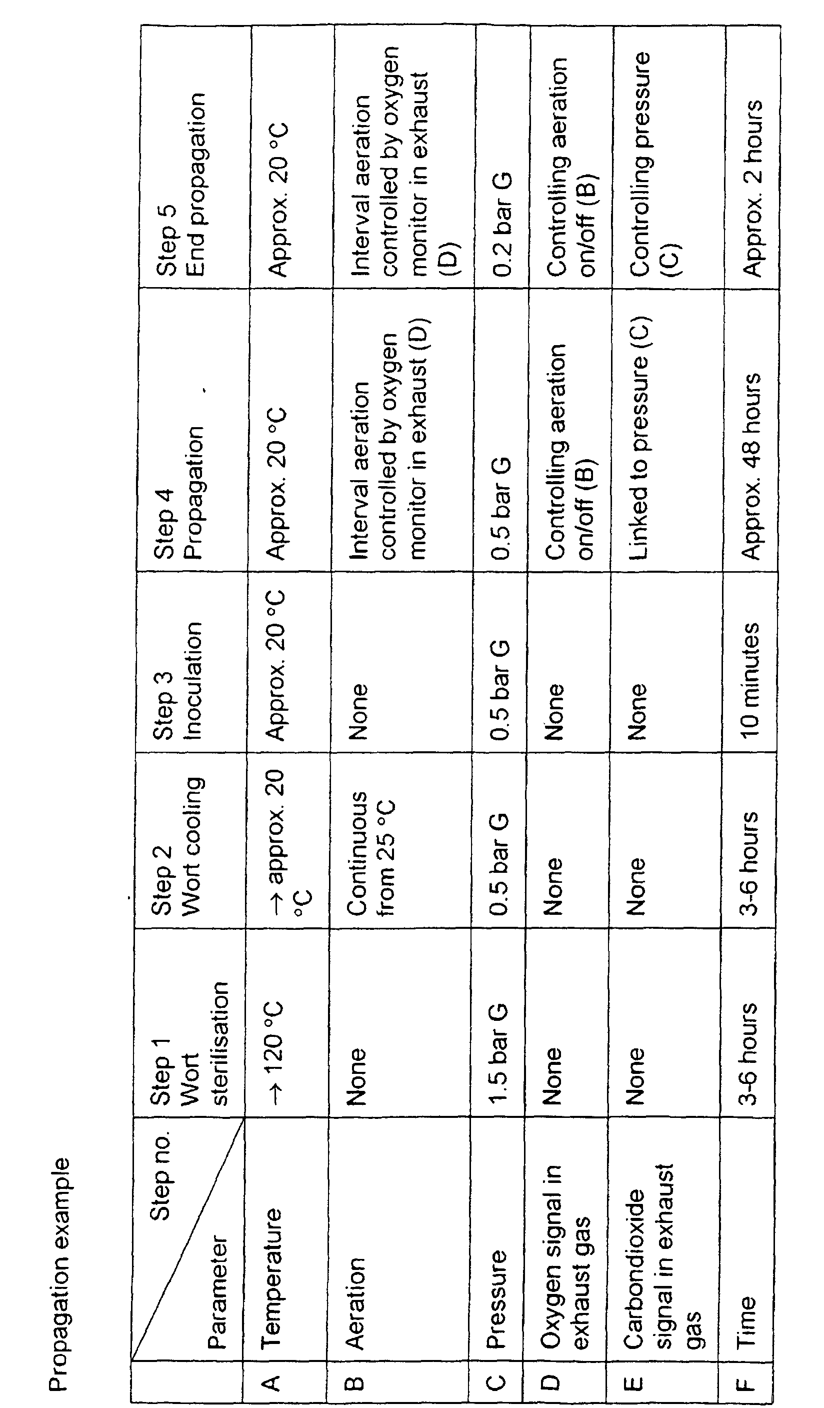

## Claims

1. A method of controlling growth of yeast for brewing beer in a nutrient medium to produce a culture of said yeast, comprising the following steps of
- adding to the nutrient medium one or more gaseous materials selected from the group consisting of oxygen or oxygen in combination with other gaseous materials which are consumed by the organism, or which do not affect the organism;
- monitoring the one or more gaseous materials, or monitoring one or more gaseous materials produced by the yeast, in the exhaust gas of the nutrient medium; and
- controlling the addition of the one or more gaseous materials in response to desired levels of the one or more of the monitored gaseous materials in the exhaust gas of the nutrient medium
wherein the oxygen content in the exhaust gas of the nutrient medium is regulated and kept close to zero, such that the yeast maintains its anaerobic fermentation characteristics after the cells have been subjected to aerobic growth.

2. A method according to claim 1, wherein the oxygen content in the exhaust gas of the nutrient medium is kept between 0 and 1 mg O₂ per liter:

3. A method according to claim 1 further comprising controlling the addition of the one or more gaseous materials in response to the temperature of the nutrient medium.

4. A method according to any of the claims 1-3 wherein the nutrient medium is contained in an essentially closed container with at least one outlet for the exhaust gas and the one or more added and/or produced gaseous materials are monitored in said at least one outlet outside the container.

5. A method according to any of the claims 1-4 wherein the nutrient medium is contained in an essentially closed container with at least one outlet for the exhaust gas and the one or more added and/or produced gaseous materials are monitored in the exhaust gas one or more locations of the headspace of the container above the nutrient medium.

6. A method according to any of the claims 4 or 5 wherein the one or more gaseous materials are monitored both in said at least one outlet of the exhaust gas outside the container and in said one or more locations of the headspace of the container above the nutrient medium.

7. A method according to any of the preceding claims wherein the addition of the one or more gaseous materials is adapted in response to a decrease and/or an increase in one or more of the monitored gaseous materials, or a derivative thereof.

8. A method according to claim 5 wherein the control of the addition of one or more gaseous materials is further adapted in response to one or more constant levels of one or more other monitored gaseous materials.

9. A method according to any of the preceding claims wherein the one or more gaseous materials which are consumed by the yeast, or which do not affect the yeast, is selected from the group consisting of oxygen, carbon dioxide, and nitrogen, and mixtures thereof.

10. A method according to any of the preceding claims wherein the response of the one or more of the monitored gaseous materials is measured in concentration of gaseous component with respect to the total gaseous material.

11. A method according to any of the preceding claims wherein the method is carried out in a batch process.

12. A method according to any of the claims 1-10 wherein the method is carried out in a continuous process.

13. A method according to any of the claims 1-12 wherein the addition of the one or more gaseous materials in response to desired levels of the one or more of the monitored gaseous materials in the exhaust gas is controlled by means comprising a computer.

14. A computer readable medium comprising program code means adapted to cause, when run on a data processing system, the data processing system to perform a method of controlling growth of a yeast for brewing beer in a nutrient medium to produce a culture of said yeast, comprising monitoring one or more added and/or produced gaseous materials in the exhaust gas of the nutrient medium, controlling the addition of one or more gaseous materials selected from the group consisting of oxygen or oxygen in combination with other gaseous materials which are consumed by the organism, or which do not affect the organism, in response to desired levels of the one or more of monitored gaseous materials in the exhaust gas in order to regulate the oxygen content in the exhaust gas of the nutrient medium and keeping it close to zero.

15. A computing system configured to perform the method of controlling growth of a yeast for brewing beer in a nutrient medium to produce a culture of said yeast, comprising monitoring one or more added and/or produced gaseous materials in the exhaust gas of the nutrient medium, controlling the addition of one or more gaseous materials selected from the group consisting of oxygen or oxygen in combination with other gaseous materials which are consumed by the organism, or which do not affect the organism, in response to desired levels of the one or more of monitored gaseous materials in the exhaust gas in order to regulate the oxygen content in the exhaust gas of the nutrient medium and keeping it close to zero.

## Patentansprüche

1. Verfahren zum Regulieren des Wachstums von Hefe zum Bierbrauen in einem Nährmedium zur Erzeugung einer Kultur der besagten Hefe, umfassend die folgenden Schritte:
Hinzufügen zu dem Nährmedium von einem oder von mehreren gasförmigen Materialien, gewählt aus der Gruppe bestehend aus Sauerstoff oder Sauerstoff in Kombination mit anderen gasförmigen Materialien, die durch den Organismus verbraucht werden oder die sich auf den Organismus nicht auswirken;
Überwachen des einen oder von mehreren gasförmigen Materialien oder Überwachen von einem oder mehreren durch die Hefe erzeugten gasförmigen Materialien im Abgas des Nährmediums; und
Regulieren der Hinzufügung des einen oder von den mehreren gasförmigen Materialien in Reaktion auf gewünschte Anteile des einen oder von den mehreren überwachten gasförmigen Materialien im Abgas des Nährmediums;
wobei der Sauerstoffgehalt in dem Abgas des Nährmediums reguliert wird und nahe null gehalten wird, sodass die Hefe ihre anaerobe Fermentierungscharakteristika beibehält, nachdem die Zellen einem aeroben Wachstum unterworfen wurden.

2. Verfahren nach Anspruch 1, wobei der Sauerstoffgehalt in dem Abgas des Nährmediums zwischen 0 und 1 mg O₂ pro Liter gehalten wird.

3. Verfahren nach Anspruch 1, weiterhin umfassend das Regulieren der Hinzufügung des einen oder von mehreren gasförmigen Materialien in Reaktion auf die Temperatur des Nährmediums.

4. Verfahren nach mindestens einem der Ansprüche 1 - 3, wobei das Nährmedium in einem im Wesentlichen geschlossenen Behälter mit mindestens einem Auslass für das Abgas enthalten ist und das eine oder mehrere hinzugefügte und/oder erzeugte gasförmige Materialien in dem mindestens einen Auslass außerhalb des Behälters überwacht werden.

5. Verfahren nach mindestens einem der Ansprüche 1 - 4, wobei das Nährmedium in einem im Wesentlichen geschlossenen Behälter mit mindestens einem Auslass für das Abgas enthalten ist und das eine oder mehrere hinzugefügte und/oder erzeugte gasförmige Materialien in dem Abgas an einer oder an mehreren Stellen des Kopfraums des Behälters über dem Nährmedium überwacht werden.

6. Verfahren nach mindestens einem der Ansprüche 4 oder 5, wobei das eine oder mehrere gasförmige Materialien sowohl in dem mindestens einen Auslass des Abgases außerhalb des Behälters als auch an der einen oder an mehreren Stellen des Kopfraums des Behälters über dem Nährmedium überwacht werden.

7. Verfahren nach mindestens einem der vorgenannten Ansprüche, wobei die Hinzufügung des einen oder von mehreren gasförmigen Materialien in Reaktion auf eine Abnahme und/oder eine Zunahme in einem oder mehreren der überwachten gasförmigen Materialien oder einem Derivat davon angepasst wird.

8. Verfahren nach Anspruch 5, wobei die Regulierung der Hinzufügung von einem oder mehreren gasförmigen Materialien weiter in Reaktion auf einen oder mehrere konstante Anteile von einem oder mehreren anderen überwachten gasförmigen Materialien angepasst wird.

9. Verfahren nach mindestens einem der vorgenannten Ansprüche, wobei das eine oder mehrere gasförmige Materialien, die durch die Hefe verbraucht werden oder die sich auf die Hefe nicht auswirken, gewählt ist/sind aus der Gruppe bestehend aus Sauerstoff, Kohlendioxid und Stickstoff und Mischungen davon.

10. Verfahren nach mindestens einem der vorgenannten Ansprüche, wobei die Antwortreaktion des einen oder von mehreren der überwachten gasförmigen Materialien in der Konzentration der gasförmigen Komponente bezüglich des gesamten gasförmigen Materials gemessen wird.

11. Verfahren nach mindestens einem der vorgenannten Ansprüche, wobei das Verfahren in einem Chargenverfahren durchgeführt wird.

12. Verfahren nach mindestens einem der Ansprüche 1 - 10, wobei das Verfahren in einem kontinuierlichen Verfahren durchgeführt wird.

13. Verfahren nach mindestens einem der Ansprüche 1 - 12, wobei die Hinzufügung des einen oder von mehreren der gasförmigen Materialien in Reaktion auf gewünschte Anteile des einen oder von mehreren der überwachten gasförmigen Materialien in dem Abgas durch Mittel, die einen Computer umfassen, reguliert wird.

14. Computerlesbares Medium, umfassend Programmcodemittel, die so angepasst sind, dass sie beim Laufen lassen auf einem Datenverarbeitungssystem das Datenverarbeitungssystem ein Verfahren zum Regulieren des Wachstums einer Hefe zum Bierbrauen in einem Nährmedium durchführen lassen, um eine Kultur der Hefe zu erzeugen, umfassend das Überwachen von einem oder mehreren hinzugefügten und/oder erzeugten gasförmigen Materialien in dem Abgas des Nährmediums, Regulieren der Hinzufügung von einem oder mehreren gasförmigen Materialien, gewählt aus der Gruppe bestehend aus Sauerstoff oder Sauerstoff in Kombination mit anderen gasförmigen Materialien, die durch den Organismus verbraucht werden oder die sich nicht auf den Organismus auswirken, in Reaktion auf gewünschte Anteile des einen oder von mehreren überwachten gasförmigen Materialien in dem Abgas, um den Sauerstoffgehalt in dem Abgas des Nährmediums zu regulieren, und dessen Halten bei nahe null.

15. Computersystem, konfiguriert für die Durchführung des Verfahrens zum Reguiieren des Wachstums einer Hefe zum Bierbrauen in einem Nährmedium, um eine Kultur der Hefe zu erzeugen, umfassend das Überwachen von einem oder mehreren hinzugefügten und/oder erzeugten gasförmigen Materialien im Abgas des Nährmediums, Regulieren der Hinzufügung von einem oder mehreren gasförmigen Materialien, gewählt aus der Gruppe bestehend aus Sauerstoff oder Sauerstoff in Kombination mit anderen gasförmigen Materialien, die durch den Organismus verbraucht werden oder die sich nicht auf den Organismus auswirken, in Reaktion auf gewünschte Anteile des einen oder von mehreren überwachten gasförmigen Materialien im Abgas, um den Sauerstoffgehalt in dem Abgas des Nänrmediums zu regulieren, und dessen Halten bei nahe null.

## Revendications

1. Méthode de régulation de la croissance de levure pour le brassage de la bière dans un milieu nutritif pour produire une culture de ladite levure, comprenant les étapes suivantes consistant à :
- ajouter au milieu nutritif une ou plusieurs substances gazeuses choisies dans le groupe constitué de l'oxygène ou de l'oxygène en combinaison avec d'autres substances gazeuses consommées par l'organisme, ou qui n'affectent pas l'organisme ;
- contrôler la ou les substances gazeuses, ou contrôler une ou plusieurs substances gazeuses produites par la levure, dans le gaz d'échappement du milieu nutritif; et
- réguler l'ajout de la ou des substances gazeuses en fonction des niveaux désirés de la ou des substances gazeuses contrôlées dans le gaz d'échappement du milieu nutritif
dans laquelle la teneur en oxygène dans le gaz d'échappement du milieu nutritif est régulée et maintenue proche de zéro, de telle façon que la levure maintienne ses caractéristiques de fermentation anaérobie après que les cellules ont été soumises à une croissance aérobie.

2. Méthode selon la revendication 1, dans laquelle la teneur en oxygène dans le gaz d'échappement du milieu nutritif est maintenu entre 0 et 1 mg d'O₂ par litre.

3. Méthode selon la revendication 1, comprenant en outre la régulation de l'ajout de la ou des substances gazeuses en fonction de la température du milieu nutritif.

4. Méthode selon l'une quelconque des revendications 1 à 3, dans laquelle le milieu nutritif est contenu dans un récipient essentiellement fermé avec au moins un orifice de sortie pour les gaz d'échappement et la ou les substances gazeuses ajoutées et/ou produites sont contrôlées dans ledit orifice de sortie à l'extérieur du récipient.

5. Méthode selon l'une quelconque des revendications 1 à 4, dans laquelle le milieu nutritif est contenu dans un récipient essentiellement fermé avec au moins un orifice de sortie pour les gaz d'échappement et la ou les substances gazeuses ajoutées et/ou produites sont contrôlées dans le gaz d'échappement en un ou plusieurs endroits de la partie vide du récipient au-dessus du milieu nutritif.

6. Méthode selon l'une quelconque des revendications 4 ou 5, dans laquelle la ou les substances gazeuses sont contrôlées à la fois dans ledit orifice de sortie de gaz d'échappement à l'extérieur du récipient et dans ledit ou lesdits endroits de la partie vide du récipient au-dessus du milieu nutritif.

7. Méthode selon l'une quelconque des revendications précédentes, dans laquelle l'ajout de la ou des substances gazeuses est adapté en fonction d'une diminution et/ou d'une augmentation d'une ou plusieurs des substances gazeuses contrôlées, ou d'un dérivé de celles-ci.

8. Méthode selon la revendication 5, dans laquelle la régulation de l'ajout d'une ou plusieurs substances gazeuses est en outre adaptée en fonction d'un ou plusieurs niveaux constants d'une ou plusieurs des autres substances gazeuses contrôlées.

9. Méthode selon l'une quelconque des revendications précédentes, dans laquelle la ou les substances gazeuses consommées par la levure, ou qui n'affectent pas la levure, sont choisies dans le groupe constitué de l'oxygène, du dioxyde de carbone, et de l'azote, et des mélanges de ceux-ci.

10. Méthode selon l'une quelconque des revendications précédentes, dans laquelle la réponse de la ou des substances gazeuses contrôlées est mesurée par la concentration du composant gazeux par rapport à la substance gazeuse totale.

11. Méthode selon l'une quelconque des revendications précédentes, dans laquelle la méthode est exécutée suivant un processus discontinu.

12. Méthode selon l'une quelconque des revendications 1 à 10, dans laquelle la méthode est exécutée suivant un processus continu.

13. Méthode selon l'une quelconque des revendications 1 à 12, dans laquelle l'ajout de la ou des substances gazeuses en fonction des niveaux désirés de la ou des substances gazeuses contrôlées dans le gaz d'échappement est régulé par des moyens comprenant un ordinateur.

14. Support lisible par un ordinateur, comprenant des moyens de codes informatiques permettant, lorsque exécutés sur un système de traitement de l'information, la mise oeuvre par le système de traitement de l'information d'une méthode de régulation de la croissance d'une levure pour le brassage de la bière dans un milieu nutritif pour produire une culture de ladite levure, comprenant le contrôle d'une ou plusieurs substances gazeuses ajoutées et/ou produites dans le gaz d'échappement du milieu nutritif, la régulation de l'ajout d'une ou plusieurs substances gazeuses choisies dans le groupe constitué de l'oxygène ou de l'oxygène en combinaison avec d'autres substances gazeuses consommées par l'organisme, ou qui n'affectent pas l'organisme, en fonction des niveaux désirés de la ou des substances gazeuses contrôlées dans le gaz d'échappement de manière à réguler la teneur en oxygène dans le gaz d'échappement du milieu nutritif et de la maintenir proche de zéro.

15. Système informatique configuré pour mettre en oeuvre la méthode de régulation de la croissance d'une levure pour le brassage de la bière dans un milieu nutritif pour produire une culture de ladite levure, comprenant le contrôle d'une ou plusieurs substances gazeuses ajoutées et/ou produites dans le gaz d'échappement du milieu nutritif, la régulation de l'ajout d'une ou plusieurs substances gazeuses choisies dans le groupe constitué de l'oxygène ou de l'oxygène en combinaison avec d'autres substances gazeuses consommées par l'organisme, ou qui n'affectent pas l'organisme, en fonction des niveaux désirés de la ou des substances gazeuses contrôlées dans le gaz d'échappement de manière à réguler la teneur en oxygène dans le gaz d'échappement du milieu nutritif et de la maintenir proche de zéro.
